# EUROPÄISCHE PATENTANMELDUNG

(11) **EP 1 018 501 A1**
(43) Veröffentlichungstag der Anmeldung: **12.07.2000**
(21) Anmeldenummer: 99124101.9
(22) Anmeldetag: 02.12.1999
(51) Int. Cl.: C07C 37/68

(54) **Verfahren zur Entfernung von organischen und/oder anorganischen Säuren aus Spaltproduktphasen**

(30) Priorität: 08.01.1999 DE 19900387
(71) Anmelder: Phenolchemie GmbH & Co. KG, 45966 Gladbeck (DE)
(72) Erfinder: Pompetzki, Werner, Dr., 46284 Dorsten (DE); Gerlich, Otto, Dr., 45966 Gladbeck (DE)

(57) **Zusammenfassung**

Es wird ein Verfahren zur Entfernung von organischen und/oder anorganischen Säuren aus Spaltproduktphasen, die bei der Spaltung von Aralkylhydroperoxiden entstehen, beansprucht, welches gekennzeichnet ist durch Extraktion der Spaltproduktphasen mittels zumindest eines Extraktionsmittels, nachfolgende Behandlung des organischen Teils der Phasen mit einem Ionenaustauscher und anschließende zumindest einmalige destillative Behandlung dieser organischen Phasen. Dabei fällt eine überwiegend wäßrige, organische Säuren aufweisende Phase an, die mit einem zweiten Ionenaustauscher behandelt wird. Der Vorteil besteht in der Entfernung von organischen Säuren aus einem wäßrigen Produktstrom, so daß weniger Austauscherkapazität bereitgestellt werden muß als bei der direkten Entfernung der organischen Säuren aus der Spaltproduktphase mittels Anionenautauscher.

Das erfindungsgemäße Verfahren wird unter anderem zur Entfernung von organischen und/oder anorganischen Säuren aus der bei der Phenolherstellung durch säurekatalysierte Spaltung von Cumolhydroperoxid anfallenden Spaltproduktphase genutzt.

## Beschreibung

Die vorliegende Erfindung betrifft ein Verfahren zur Entfernung von organischen und/oder anorganischen Säuren aus Phasen, die bei der Spaltung von Aralkylhydroperoxiden entstehen.

Im besonderen betrifft die vorliegende Erfindung ein Verfahren zur Entfernung von organischen und/oder anorganischen Säuren aus Phasen, die bei der Spaltung von Cumolhydroperoxid entstehen.

Bei vielen säurekatalysierten Prozessen verbleibt ein nicht unerheblicher Teil der als Katalysator eingesetzten organischen und/oder anorganischen Säuren in den entstehenden Gemischen. Die Gemische werden, wenn möglich, durch Phasentrennung in eine wäßrige und eine organische Phase getrennt, wobei in der organischen Phase immer noch anorganische Bestandteile wie z. B. Wasser und anorganische Säuren gelöst sind. Bei der Aufarbeitung dieser Produktphase kann die noch vorhandene Säure unerwünschte Nebenreaktionen wie z. B. säurekatalysierte Alkylierungs- oder Kondensationsreaktionen hervorrufen, die zu Ausbeuteverlusten an Wertprodukt führen. Um solche unerwünschten Nebenreaktionen zu unterbinden, ist es deshalb notwendig, daß die organischen und/oder anorganischen Sauren aus der organischen Phase möglichst schnell abgetrennt werden.

Ein Prozeß, der von großer wirtschaftlicher Bedeutung ist, ist der bei der Gewinnung von Phenol und Aceton ausgehend von Cumol nach dem klassischen Hock-Verfahren durchgeführte Prozeß der säurekatalysierten Spaltung von Cumolhydroperoxid zu Phenol und Aceton. Bei diesem Prozeß fällt bei der durch Schwefelsäure katalysierten Spaltreaktion von Cumolhydroperoxid (CHP) zu Phenol und Aceton eine saure, überwiegend organische Phase an, welche Schwefelsäure enthalt. Hinzu kommen in der Regel organische Säuren, welche als Nebenprodukte bei der Oxidation von Cumol oder bei der säurekatalysierten Spaltung entstehen können, so daß der Gesamtsäuregehalt noch etwas höher liegt. Zur Vermeidung von säurekatalysierten Alkylierungs- und Kondensationsreaktionen im Spaltprodukt und zur Vermeidung von Korrosionsschäden an den eingesetzten Apparaten müssen vor der beispielsweise auf destillativem Wege erfolgenden Trennung des Spaltprodukts in seine Bestandteile die Säuren schnell aus dem Spaltprodukt entfernt werden.

Dies geschieht praktisch üblicherweise durch sofortige Neutralisation des bei der säurekatalysierten Spaltung von Cumolhydroperoxid entstehenden Spaltprodukts mit wäßrigen Lösungen von Alkalihydroxiden und/oder -phenolaten und anschließende Salzwäsche zur Entfernung des anfallenden Alkalisulfats (so beschrieben in EP 0 032 255, EP 0 085 289, BP 756 408, DE 1 128 859 und DE 25 12 842). Trotz Neutralisation und Salzwäsche bleibt jedoch noch eine nicht vernachlässigbare Menge an Alkalisulfat bzw. Alkalicarboxylaten im Spaltprodukz gelöst und oder emulgiert. In der nachgeschalteten Destillation kommt es deswegen immer weider zu Salzablagerungen, die ein regelmäßiges Spülen und Reinigen der Verdampfer erfordern. Der hohe Salzgehalt, der bei der Destillation im Sumpf der Destillationskolonne zurückbleibt, erschwert außerdem die Aufarbeitung des Rückstands, der bei der Destillation zurückbleibt, weshalb es notwendig ist, die Salze möglichst früh aus dem Spaltproduktgemisch abzutrennen.

Aus US 4 262 150 und US 4 262 151 ist weiterhin bekannt, daß durch gezielte Neutralisation des bei der säurekatalysierten Spaltung von Cumolhydroperoxid entstehenden Spaltproduktes unter Einhaltung eines pH-Wertes von 2 bis 6 und anschließende Salzwäsche eine Verbesserung der Salzabtrennung erzielt werden kann.

In US 5 510 543 wird eine weitere Möglichkeit zur Verbesserung der Salzabtrennung beschrieben. Diese wird erreicht durch Zugabe von 3 bis 5 Gew.-% Cumol zum Spaltproduktgemisch vor der Neutralisation.

Die Neutralisation des bei der säurekatalysierten Spaltung von Cumolhydroperoxid entstehenden Spaltproduktgemisches ist, wie in US 3 927 124 beschrieben, auch durch die Verwendung von Ammoniak, Methylamin oder Triethylamin möglich.

Die nach der Neutralisation notwendige Salzwäsche wird bei all diesen Verfahren oft dadurch erschwert, daß es bei der Salzwäsche zu nicht unerheblichen Phasentrennungsproblemen kommen kann.

Aus der Patentschrift DE 963 520 ist daher auch die Entfernung der Schwefelsäure aus dem bei der säurekatalysierten Spaltung von Cumolhydroperoxid entstehenden sauren Spaltprodukt durch die Extraktion mit einer schwachen nicht flüchtigen Carbonsäure bekannt.

In DD 91 643 ist die Entfernung der Schwefelsäure aus dem bei der säurekatalysierten Spaltung von Cumolhydroperoxid entstehen sauren Spaltprodukt durch Extraktion mit Wasser beschrieben. Dabei sind jedoch erhebliche Wassermengen (15 bis 35 Gew.-%) erforderlich, um den Schwefelsäuregehalt auf unter 10 ppm im Spaltprodukt zu senken, die anschließend bearbeitet werden müssen, bevor sie dem Abwasser zugeführt werden können. Die Extraktion erfolgt in einer Extraktionskolonne mit 10 mm großen Raschigringen bei Temperaturen von 20 bis 40 °C. Dieses Verfahren ist auch auf ein teilneutralisiertes Spaltprodukt anwendbar.

Aus CA 697 600, FR 1 302 848 und SU 118 415 ist außerdem bekannt, daß die Entfernung von Schwefelsäure aus dem bei der säurekatalysierten Spaltung von Cumolhydroperoxid entstehenden Spaltprodukt ohne Extraktion durch den Einsatz von Anionenaustauschern möglich ist, wobei die Regeneration des Anioneraustauschers mit einer Alkalihydroxidlösung erfolgt. Der Einsatz der Ionenaustauscherharze zur Entfernung der Schwefelsäure aus dem Spaltprodukt bedingt jedoch auf Grund der geringen Beständigkeit der Anionenaustauscherharze gegenüber dem Spaltprodukt, eine verhältnismäßig kurze Lebensdauer des Anionenaustauschers.

In DE 198 35 306 ist ein Verfahren zur Entfernung von orzanischen und oder anorganischen Säuren aus organischen Phasen beschrieben, welches die organischen und/oder anorganischen Säuren mittels Extraktion und anschließender Behandlung der organischen Phase mit einem Ionenaustauscher aus der organischen Phase entfernt. Beim Einsatz dieses Verfahrens sind große Ionenaustauschermengen notwendig, so daß beim Betrieb (Regeneration, Spülung und Austausch) des Ionenaustauscherharzes erhebliche Kosten entstehen.

Der Erfindung liegt daher die Aufgabe zugrunde, ein einfaches und kostengünstiges Verfahren zur Entfernung von organischen und/oder anorganischen Säuren aus Spaltproduktphasen bereitzustellen.

Im besonderen liegt der Erfindung die Aufgabe zugrunde, ein einfaches Verfahren zur Entfernung von organischen und/oder anorganischen Säuren aus Phasen, die bei der Spaltung von Aralkylhydroperoxiden wie z. B. Cumolhydroperoxid entstehen, bereitzustellen, das die Herstellung eines salzarmen bzw. -freien und damit leichter aufarbeitbaren Spaltprodukts ermöglicht.

Es wurde nun überraschenderweise gefunden, daß die Entfernung von organischen und/oder anorganischen Säuren aus Phasen, die bei der Spaltung von Aralkylhydroperoxiden entstehen, durch Extraktion, eine anschließende Behandlung der organischen Phase mit einem Ionenaustauscher, sowie durch eine destillative Behandlung der organischen Phase und eine Behandlung einer, bei der destillativen Behandlung von der organischen Phase abgetrennten, zumindest eine organische Säure aufweisenden überwiegend wäßrigen Phase mit einem zweiten Ionenaustauscher und/oder durch Hinzufügen eines Phenolstroms zur Spaltproduktphase vor der Extraktion wesentlich verbessert wird und wirtschaftlicher durchzuführen ist.

Gegenstand der vorliegenden Erfindung ist daher ein Verfahren zur Entfernung von organischen und/oder anorganischen Säuren aus Spaltproduktphasen, die bei der Spaltung von Aralkylhydroperoxiden entstehen, durch Extraktion der Spaltproduktphasen mittels zumindest eines Extraktionsmittels und nachfolgender Behandlung des organischen Teils der Phasen mit einem Ionenaustauscher, dadurch gekennzeichnet. daß die mit einem Ionenaustauscher behandelten organischen Phasen zumindest einmal destillativ behandelt werden, bei dieser Behandlung zumindest eine überwiegend wäßrige, zumindest eine organische Säure aufweisende Phase von den organischen Phasen abgetrennt wird und diese Phase mit einem zweiten Ionenaustauscher behandelt wird.

Ebenfalls Gegenstand der vorliegenden Erfindung ist ein Verfahren zur Entfernung von organischen und/oder anorganischen Säuren aus Spaltproduktphasen, die bei der Spaltung von Aralkylhydroperoxiden entstehen, durch Extraktion der Spaltproduktphasen mittels zumindest eines Extraktionsmittels und nachfolgender Behandlung des organischen Teils der Phasen mit einem Ionenaustauscher, dadurch gekennzeichnet, daß der Spaltproduktphase vor der Extraktion zumindest ein alkalischer Phenolstrom zugefügt wird.

Mit Hilfe des erfindungsgemäßen Verfahrens können aus organische und oder anorganische Säuren und anorganische Bestandteile enthaltenden organischen Phasen überraschend gut anorganische Bestandteile und Säuren entfernt werden. Ein Vorteil gegenüber den bekannten Verfahren besteht darin, daß im Extraktionsschritt nur etwa ein Viertel der Menge an Extraktionsmittel benötigt wird als bei den herkömmlichen Verfahren, da eine vollständige Entfernung der Säuren, wie dies bei den herkömmlichen Verfahren notwendig ist, nicht nötig ist. Wird im Extraktionsschritt Wasser als Extraktionsmittel eingesetzt, so erhält man im Vergleich zu den herkömmlichen Verfahren eine weniger verdünnte Schwefeisäure-Lösung, die aufgrund der Konzentration im Prozeß verwendet werden kann. Zur Extraktion wird vorzugsweise soviel Wasser eingesetzt, daß die zu extrahierende Phase mit Wasser gesättigt ist und zusätzlich bis zu 7 Gew.-%, vorzugsweise von 2 bis 7 Gew.-% und ganz besonders bevorzugt von 2 bis 4 Gew.-% Wasser, bezogen auf die Phase, zur Extraktion verwendet werden. Ein zusätzlicher wichtiger Effekt dieser Extraktion ist die weitgehende Entfernung aller, aus den vorhergehenden Prozeßschritten eingetragener Salze. Der in der organischen Phase verbleibende Rest an Schwefelsäure wird mit einem handelsüblichen schwach basischen Ionenaustauscher entfernt. Die Verwendung eines Ionenaustauschers hat den Vorteil daß nicht nur der Anteil an anorganischen Säuren sondern auch ein Teil an organischen Säuren, insbesondere Ameisen- und/oder Essigsäure, in der organischen Phase sehr effektiv verringert wird. In einer besonders bevorzugten Ausfürungsart des Verfahrens im Zuge der Phenolherstellung aus Cumol kann zur Regeneration des eingesetzten Ionenaustauschers z.B. Phenolatlauge eingesetzt werden. Diese fällt im Gesamtprozeß der Phenolherstellung nach dem Cumolverfahren beim Auswaschen von Phenol aus dem Kopfprodukt der Cumol-Kolonne, welches hauptsächlich Cumol und α-Methylsryrol enthält, mit Alkalilauge an. Durch Einsatz der Phenolatlauge zur Regeneration des Anionentauschers kann der Verbrauch von reinem Alkalihydroxid, welches üblicherweise zur Regeneration eines Anionenaustauschers eingesetzt wird, vermieden werden und somit ein erheblicher Kostenvorteil erzielt werden. Zusätzlich wird die Menge des Phenolats in der Phenolatlauge, die zur Rückgewinnung des Phenols angesäuert werden muß, geringer, da im Ionentauscher die Säure gegen das Phenol getauscht wird und somit schon ein Teil des Phenols zurückgewonnen wird. Dadurch, daß der organischen Phase vor der Behandlung im Ionenaustauscher ein großer Teil der Säuren durch Extraktion entzogen wird, wird der Ionenaustauscher, welcher nur zur Nachreinigung der organischen Phase eingesetzt wird, geringer belastet und hat damit eine längere Lebensdauer, welche sich positiv auf die Betriebskosten auswirkt.

Ein weiterer Vorteil des erfindungsgemäßen Verfahrens liegt darin, daß das Volumen des schwach basischen Anionenaustauschers relativ klein gehalten werden kann, da es nicht notwendig ist, die organischen Säuren restlos aus der organischen Phase zu entfernen. Die organischen Säuren, insbesondere Ameisen- und/oder Essigsäure, fallen bei der weiteren Aufarbeitung der Spaltproduktphase, insbesondere bei der Abtrennung von Cumol und α-Methylstyrol von der Spaltproduktphase in einer überwiegend Wasser enthaltenden Phase an. Aus dieser Phase lassen sich die organischen Säuren relativ einfach abtrennen, in dem die Phase mit einem stark basischen Anionenaustauscher behandelt wird. Dieser Anionenaustauscher muß zwar mit Natronlauge regeneriert werden, kann aber, da die organischen Säuren aus einer wäßrigen Phase entfernt werden, relativ klein gehalten werden. Vor diesem Hintergrund erfordert der Betrieb (Regeneration, Spülung und Austausch) des Ionenaustauschers einen erheblich geringeren technischen und finanziellen Aufwand.

Durch die erfindungsgemäße Zugabe eines alkalischen Phenolstroms zur Spaltproduktphase vor der Extraktion der Spaltproduktphase werden geringe Konzentrationen der in der Spaltproduktphase vorhandenen Säuren neutralisiert und können als Salz aus der organischen Phase extrahiert werden. Das durch den Phenolstrom eingetragene Phenol verbleibt in der organischen Phase und wird bei der Aufarbeitung der organischen Spaltproduktphase zurückgewonnen. Durch diese besondere Ausführungsart des erfindungsgemäßen Verfahrens läßt sich zum einen die Abtrennung der Säuren verbessern und zum anderen läßt sich Phenol, welches in einem alkalischen Phenolstrom im Aufarbeitungsprozeß der Spaltproduktphase anfällt und bis jetzt aufwendig aufgearbeitet oder verworfen werden mußte, wieder in den Aufarbeitungsprozeß zurückführen.
Das erfindungsgemäße Verfahren wird im folgenden beispielhaft anhand der Spaltung von Cumolhydroperoxid, welches ein Zwischenprodukt der Hock'schen Phenolsynthese ist, beschrieben, ohne darauf beschränkt zu sein.

Bei dieser in bekannter Weise durchgeführten Synthese wird aus Cumol und Sauerstoff Cumolhydroperoxid hergestellt. Dieses wird in einer Spaltreaktion säurekatalysiert in Phenol und Aceton aufgespalten. Als Katalysator wird Säure, zum Beispiel Schwefelsäure, verwendet. Die weitere beispielhafte Beschreibung des erfindungsgemäßen Verfahrens bezieht sich auf die Durchführung der Spaltreaktion mit einem Einsatz von Säure als Katalysator, ohne jedoch darauf beschränkt zu sein.

Die saure Spaltproduktphase, welche neben den Spaltprodukten und Nebenprodukten die als Katalysator verwendete Säure sowie organische Säuren, welche als Nebenprodukte bei der Oxidation von Aralkylen, wie z.B. Cumol, gebildet werden, enthalten kann, wird zum Entfernen der Säuren in einem ersten Schritt des erfindungsgemäßen Verfahrens in eine Extraktionsvorrichtung, vorzugsweise eine Extraktionskolonne gefahren. Die Einspeisung der sauren Spaltproduktphase in eine flüssig-flüssig Extraktionskolonne erfolgt vorzugsweise unterhalb des Kopfes, besonders bevorzugt unmittelbar oberhalb des Sumpfes. Die Extraktionskoionne wird oberhalb des Sumpfes der Kolonne, vorzugsweise unmittelbar unterhalb des Kopfes der Kolonne mit einem Extraktionsmittel beschickt. Als Extraktionsmittel wird vorzugsweise Wasser oder ein überwiegend Wasser enthaltendes Gemisch eingesetzt. Die Einspeisung an Extraktionsmittel und zu extrahierender Phase in die Extraktionskolonne wird so gesteuert, daß bis zu 14 Gew.-% des Extraktionsmittels, vorzugsweise Wasser oder ein überwiegend Wasser enthaltendes Gemisch, bezogen auf das Spaltproduktgemisch in die Extraktionskolonne gefahren werden. Vorzugsweise wird soviel Wasser oder ein überwiegend Wasser enthaltendes Gemisch eingesetzt, daß die zu extrahierende Spaltproduktphase mit Wasser gesättigt ist und zusätzlich bis zu 7 Gew.-%, vorzugsweise von 2 bis 7 Gew.-% und ganz besonders bevorzugt von 2 bis 4 Gew.-% Wasser, bezogen auf die Spaltproduktphase, zur Extraktion verwendet werden. Das Extraktionsmittel und die zu extrahierende Phase durchströmen die Extraktionskolonne im Gleich- oder Gegenstrom, vorzugsweise im Gegenstrom.

In einer besonders bevorzugten Ausführungsart des erfindungsgemäßen Verfahrens wird der Spaltproduktphase, die bei der Spaltung von Cumolhydroperoxid erhalten wird, vor dem Einspeisen in die Extraktionsvorrichtung ein Phenol aufweisendes Gemisch zugesetzt, welches bei der Aufarbeitung des Phenols anfällt und welches die gesamte Natronlauge, die während der destillativen Aufarbeitung des Phenols dem Prozess zugegeben wurde, enthält. Während der Extraktion bilden sich aus den im Spaltprodukt vorliegenden Säureanionen mit den Natriumionen aus dem Phenol aufweisenden Gemisch Salze, welche sich nach der Extraktion überwiegend in der wäßrigen Phase befinden. Auf diese Weise ist es möglich, durch die Extraktion nicht nur die Säurekonzentration in der organischen Spaltproduktphase zu verringern, sondern auch die Konzentrationen an Salzen, wie z.B. Na₂SO₄ zu verringern. Je nach Anfangskonzentration an Salz bzw. Säure im Spaltproduktgemisch und Konzentration an NaOH und Menge des hinzugefügten Phenol aufweisenden Gemisches lassen sich Na₂SO₄-Konzentrationen von 1 bis 2 ppm in der organischen Spaltproduktphase erreichen.

Das mit Säure beladene Extraktionsmittel, bevorzuzt Wasser, wird erfindungsgemäß aus einer Extraktionsvorrichtung, vorzugsweise aus dem unteren Teil einer Extraktionskolonne, abgezogen. Im Fall von Schwefelsäure als Katalysator wird aus dem unteren Teil der Extraktionskolonne verdünnte Schwefelsäure abgezogen, die im Prozeß weiter verwendet werden kann.

Die Temperatur in der Extraktionsvorrichtung liegt zwischen 0 und 90 °C, bevorzugt zwischen 20 und 50 °C und besonders bevorzugt zwischen 30 und 40 °C.

Der aus der Extraktionsvorrichtung, vorzugsweise aus dem Kopf einer Extraktionskolonne erhaltene an wasserlöslichen Ionen abgereicherte Teil der organischen Spaltproduktphase, welche noch organische Säuren sowie eine geringe Menge anorganischer Säuren enthält, kann erfindungsgemäß über zumindest einen handelsüblichen, vorzugsweise schwach basischen Anionenaustauscher, wie z.B. Amberlyst A21 oder IRA 96 SB der Firma Rohm und Haas geleitet werden. Die Geschwindigkeit der Beladung des Anionenaustauschers hängt von der Dimensionierung des Austauscherbettes ab, welche wiederum von den zu behandelnden Volumenströmen und deren Säurekonzentrationen abhängig ist, und kann mittels Ventilen oder ähnlichem eingestellt werden.

In einer weiteren besonders bevorzugten Ausführungsart des erfindungsgemäßen Verfahrens kann der aus der Extraktionsvorrichtung erhaltene, an wasserlöslichen Ionen abgereicherte Teil der organischen Spaltproduktphase vor dem Behandeln mit einem Anionenaustauscher zur Entfernung von emulgiertem Wasser zuerst durch einen Koaleszer gefahren werden. Mit dem Koaleszer kann der Wassergehalt der organischen Spaltproduktphase und die Salzfracht weiter verringert werden. Die mit dem Koaleszer abgetrennt wäßrige Phase kann einer Aufarbeitung zugeführt werden. Soll das Wasser entsorgt werden, so müssen die eventuell vorhandenen organischen Bestandteile, wie z.B. Aceton und/oder Phenol, abgetrennt werden.

Nach dem Durchlaufen der Extraktionsvorrichtung und des Anionenaustauschers ist die organische Spaltproduktphase so weitgehend von anorganischen Säuren befreit daß sich anorganische Säuren analytisch nicht mehr nachweisen lassen, und kann einer Aufarbeitung, vorzugsweise einer destillativen Aufarbeitung, zugeführt werden. Der Salzgehalt der organischen Spaltproduktphase ist nach Durchlaufen der Extraktionsvorrichtung mit eventuell verwendetem Koaleszer und/oder des Anionenaustauschers wesentlich verringert worden und beträgt vorzugsweise von 1 bis 2 ppm, berechnet als Natriumsulfat.

Bei der destillativen Aufarbeitung der organischen Spaltproduktphase, die auf allgemein bekannte Weise erfolgen kann, werden in mehreren destillativen Schritten höher und niedriger siedende Verbindungen vom eigentlichen Produkt bei der Spaltung von Cumolhydroperoxid, vom Phenol, abgetrennt. Im Verlauf dieser destillativen Schritte fallen auch überwiegend wäßrige Phasen an, die organische Säuren wie z.B. Ameisensäure oder Essigsäure aufweisen können. Neben diesen Säuren können die überwiegend wäßrigen Phasen auch geringe Mengen organischer Produkte oder Nebenprodukte der Aralkyhydroperoxidspaltung, wie z.B. Phenol oder Cumol, aufweisen.

Es kann vorteilhaft sein, diese Phasen in die destillative Aufarbeitung zurückzufarhren, damit sich einige Bestandteile, wie z.B. die organischen Säuren, anreichern. Diese Säuren fallen wiederum in den überwiegend wäßrigen Phasen an.

Ebenso kann es vorteilhaft sein, diese anfallenden wäßrigen Phasen in einen Behäher zu leiten, in welchem organische Bestandteile von der überwiegend wäßrigen Phase abgetrennt werden können.

Die anfallenden wäßrigen, organische Säuren, wie z.B Ameisen- und/oder Essigsäure aufweisenden Phasen, die noch Produkte oder Nebenprodukte aus der Aralkylhydroperoxidspaltung, wie z.B. Phenol enthalten können, können zur Extraktion der Schwefelsäure aus dem Spaltproduktgemisch verwendet werden.

In einer besonderen Ausführungsart des erfindungsgemäßen Verfahrens können die anfallenden wäßrigen, organische Säuren wie z.B. Ameisen- und/oder Essigsäure aufeisenden Phasen, die noch Produkte oder Nebenprodukte aus der Aralkylhydroperoxidspaltung, wie z.B. Phenol enthalten können, mit einem zweiten, vorzugsweise stark basischen, Anionenaustauscher aufbereitet werden. Als Anionenaustauscher kann jeder handelsübliche Anionenaustauscher, vorzugsweise ein stark basischer Anionenaustauscher, wie z.B. Amberjet 4400 OH der Firma Rohm und Haas verwendet werden.

Neben Extraktionskolonnen kann grundsätziich (unabhängig vom Beispiel) jede kontinuierlich oder diskontinuierlich arbeitende Extraktionsvorrichtung eingesetzt werden. Vorzugsweise werden mit geeigneten Einbauten ausgestattete Extraktionskolonnen verwendet.

Als schwach basische Anionenaustauscher können handelsübliche Anionenaustauscher, wie z.B. Polyacrylamid- bzw. Styroldivinylbenzolharze, wie z.B. Amberlyst A 21 oder IRA 96 SB der Firma Rohm und Haas, verwendet werden. Als stark basische Anionenaustauscher können handelsübliche Anionenaustauscher verwendet werden, wie z.B. Amberlyst 4400 OH der Firma Rohm und Haas.

Die erfindungsgemäße Regeneration der Anionenaustauscher kann mit Lauge, z.B. mit Alkalihydroxid-Lösung, erfolgen. Zum Zwecke der Regeneration wird die Regenerationslösung in gleicher Richtung oder in entgegengesetzter Richtung zur Richtung, in welcher die zu behandelnde Phase den Anionenaustauscher durchströmt, vorzugsweise in gleicher Richtung, über den Anionenaustauscher gefahren. In einer besonders bevorzugten Ausführung des erfindungsgemäßen Verfahrens wird zur Regeneration des schwach basischen Anionenaustauschers freies Phenol enthaltende Phenolatlaugelösung, welche bei der Aufarbeitung von Phenol enthaltenden Phasen anfällt, genutzt.

Nach der Regeneration können die Anionenaustauscher mit Wasser gespült werden, das Spülen erfolgt vorzugsweise solange, bis im Spülwasser keine OH⁻-Ionen bzw. kein Alkali oder Na-Phenolat mehr nachweisbar sind.

Je nachdem, ob das Anionenaustauscherharz mit Alkalihydroxidlauge, z.B. mit NaOH, oder mit Phenolatlauge regeneriert wurde, entstehen im Anionenaustauschprozeß aus den in der organischen Phase enthaltenen Säuren Wasser oder Phenol gemäß der Formeln:
- Ionenaustauschprozeß: Harz-OH + HX → Harz-X + H₂O
- Regeneration: Harz-X + NaOH → Harz-OH + NaX
- Ionenaustauschprozeß: Harz-Phenolat + HX → Harz-X + Phenol
- Regeneration: Harz-X + Na-Phenolat → Harz-Phenolat + NaX

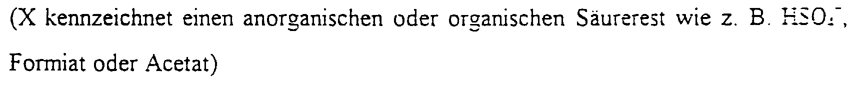

Entsteht im Ionenaustauschprozeß Wasser, so kann dieses durch z. B. einen Phasenabscheider von der organischen Phase getrennt werden. Entsteht im Ionenaustauschprozeß des besonders bevorzugten Verfahrens Phenol, so kann dieses direkt mit der organischen Spaltproduktphase der Aufarbeitung zugeführt werden.

Um das erfindungsgemäße Verfahren kontinuierlich betreiben zu können, kann es vorteilhaft sein, jeweils zumindest zwei schwach basische und zwei stark basische Anionenaustauscher einzusetzen. Diese werden vorzugsweise in der Weise angeordnet, daß während der Beladung des einen Anionenaustauschers der andere in der oben beschriebenen Weise regeneriert werden kann und umgekehrt. Das Umstellen der Anionenaustauscher von Regeneration auf Ionenaustausch und umgekehrt, kann mit Hilfe von Ventilen, Absperrhähnen oder ähnlichem auf eine dem Fachmann bekannten Weise erfolgen.

Erfindungsgemäß kann die Verwendung mehrerer Extraktionskolonnen oder mehrerer Ionenaustauscher oder die Verwendung mehrerer Kombinationen von Extraktionskolonnen mit nachgeschalteten Ionenaustauschern vorgesehen werden.

Die erfindungsgemäße Extraktion wird vorzugsweise bei Umgebungsdruck und bei Temperaturen zwischen 0 und 90 °C, bevorzugt bei Temperaturen zwischen 20 und 50 °C und besonders bevorzugt bei Temperaturen zwischen 30 und 40 °C ausgeführt.

Erfindungsgemäß kann als Extraktionsmittel saures oder neutrales Wasser, vorzugsweise neutrales Wasser verwendet werden. Vorzugsweise wird als Extraktionsmittel die wäßrige Phase eingesetzt, die aus dem Behälter nach der destillativen Aufarbeitung der Spaltproduktphase stammt und die mit Hilfe des stark basischen Ionenaustauschers von organischen und/oder anorganischen Säuren weitgehend befreit wurde.

Das erfindungsgemäße Verfahren kann kontinuierlich oder diskontinuierlich ausgeführt werden.

Es kann vorteilhaft sein bevorzugte Ausführungsarten des erfindungsgemäßen Verfahrens zu kombinieren.

Das erfindungsgemäße Verfahren ist geeignet, organische und/oder anorganische Säuren aus Phasen zu entfernen, die bei der Spaltung von Aralkylhydroperoxiden entstehen. Das erfindungsgemäße Verfahren ist besonders geeignet, organische und/oder anorganische Säuren aus Phasen zu entfernen, die bei der Spaltung von Cumolhydroperoxiden entstehen.

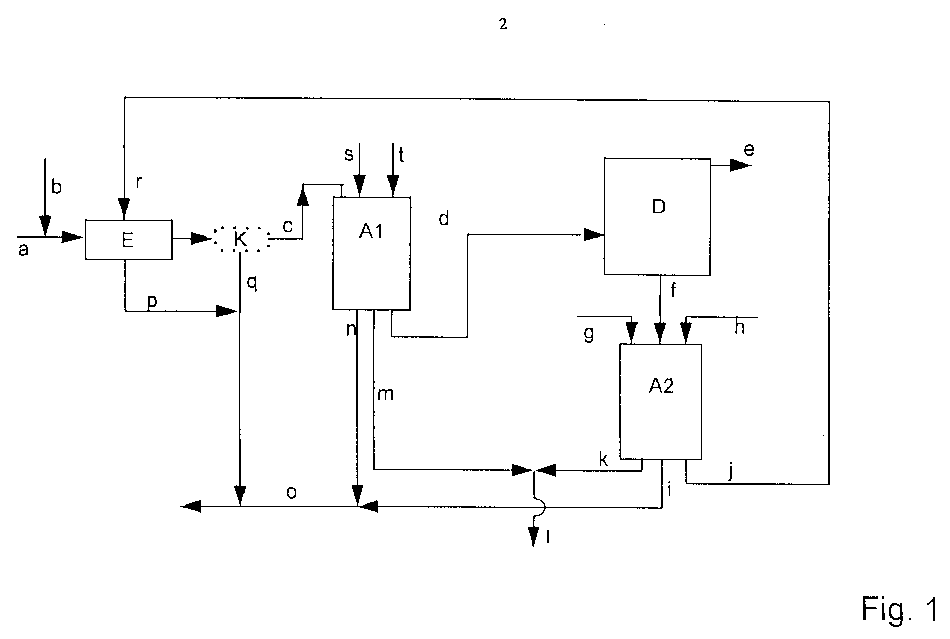

In Figur 1 ist beispielhaft eine Ausführungsart des erfindungsgemäßen Verfahrens dargestellt, ohne daß das Verfahren auf diese beschränkt ist.

Das zu behandelnde Spaltprodukt gelangt über Leitung **a** in die Extraktionsvorrichtung **E**. Über Leitung **b** kann dem Spaltprodukt vor dem Überleiten in die Extraktionsvorrichtung ein Phenolstrom zugegeben werden. Die organische Phase wird aus der Extraktionsvorrichtung über Leitung **c** über den Anionenaustauscher **A1** geleitet. In dieser Leitung kann ein Koaleszer **K** zur Abtrennung von in der organischen Phase emulgiertem Wasser vorgesehen werden. Die in der Extraktionsvorrichtung anfallende wäßrige Phase sowie die, falls vorhanden, im Koaleszer anfallende wäßrige Phase wird über Leitung **p** bzw. **q** gesammelt.

Die im Anionenaustauscher **A1** behandelte Phase wird über Leitung **d** in eine Destillationseinheit **D** geleitet, die aus mehreren Destillationskolonnen besteht. In dieser Destillationseinheit erfolg die Aufarbeitung des Spaltproduktes. Dabei fällt eine überwiegend wäßrige Phase an, die dem Anionenaustauscher **A2** über Leitung **f** zugeführt wird. Die organischen Phasen werden z.B. über Leitung **e** einer weiteren Aufarbeitung zugeführt.

Die im Anionenaustauscher **A2** behandelte Phase verläßt diesen über Leitung **j** und wird über diese Leitung zur Extraktionsvorrichtung **E** geführt und in diese eingespeist.

Über Leitung **g** kann der Anionenaustauscher **A2** mit Lauge regeneriert werden. Über Leitung **s** kann der Anionenaustauscher **A1** mit Lauge regeneriert werden. Die Regenerationslauge verläßt den Anionenaustauscher **A2** über Leitung **k** und den Anionenaustauscher **A1** über Leitung m. Beide Leitungen vereinigen sich in Leitung **1**, über die die Regenerationslauge einer Entphenolung zugeführt wird. Über die Leitungen **h** und **t** kann den Anionenaustauschern **A1** und **A2** Spülwasser zugerführt werden. Dieses Spülwasser verläßt die Anionenaustauscher **A1** und **A2** über die Leitungen **i** und **n**, die sich vereinigen zur Leitung **o**. Mit dieser Leitung **o** vereinigt sich auch Leitung **q**, die die gesammelten, in der Extraktionsvorrichtung **E** und/oder dem Koaleszer **K** angefallenen wäßrigen Phasen führt. Leitung **o** führt diese Abwässer, die Phenol und/oder Aceton enthalten können, einer Entphenolung zu.

### Beispiel 1:

### Entfernung von Schwefelsäure und organischen Säuren aus Gemischen, die bei der einphasigen Spaltung von Cumolhydroperoxid entstehen

Bei der Gewinnung von Phenol und Aceton ausgehend von Cumol nach dem klassischen Hock-Verfahren fällt bei der durch Schwefelsäure katalysierten Spaltreaktion von Cumolhydroperoxid zu Phenol und Aceton eine saure Spaltproduktphase an, welche in dem ausgeführten Beispiel ca. 950 ppm Schwefelsäure enthält. Hinzu kommen organische Säuren, so daß der Gesamtsäuregehalt (berechnet als Schwefelsäure) im Durchschnitt ca. 1200 ppm beträgt.

Die Spaltproduktphase wurde mit durchschnittlich 250 l/h durch eine Extraktionskolonne gefahren. Zusätzlich zur mit Wasser gesämigten Spaltproduktphase wurden 4 Gew.-% Wasser in die Extraktionsanlage gefahren. Nach erfolgter Extraktion bei einer Temperatur von durchschnittlich 30 °C betrug der Schwefelsäuregehalt in der organischen Phase < 20 ppm. Der Gesamtsäuregehalt in der organischen Phase betrug nach dieser Behandlung noch durchschnittlich 250 ppm (berechnet als Schwefelsäure), da sich die organischen Säuren kaum extrahieren ließen.

Diese organische Phase wurde mit 225 l/h über ein Anionenaustauscherbett, das mit 15,3 l des handelsüblichen schwach basischen Anionenaustauscherharz Amberlyst A 21 der Firma Rohm und Haas gefüllt war, gefahren. Die Temperatur der organischen Phase betrug ca. 30 °C. Nach dem Durchlaufen des Anionentauschers wurde die behandelte organische Phase analysiert und es ließ sich eine Gesamtsäurekonzentration von < 250 ppm, berechnet als H₂SO₄, in der organischen Phase feststellen. Der Beitrag der Schwefelsäure zur Gesamtsäurekonzentration betrug < 1 ppm.

### Beispiel 2:

### Entfernung von Schwefelsäure und organischen Säuren aus Gemischen, die bei der einphasigen Spaltung von Cumolhydroperoxid entstehen

Eine Spaltproduktphase wird wie in Beispiel 1 durch eine Extraktionskolonne gefahren mit dem Unterschied, daß der Spaltproduktphase zusätzlich ein alkalischer Phenolstrom mit durchschnittlich 8 l/h zugemischt wurde. Nach erfolgter Extraktion bei einer Temperatur von ca 30 °C betrug der Schwefelsäuregehalt in der organischen Phase von 100 bis 170 ppm. Der Gesarutsäuregehalt in der organischen Phase betrug nach dieser Behandlung noch durchschnittlich 300-400 ppm (berechnet als Schwefelsäure), da sich die organischen Säuren kaum extrahieren ließen.

### Beispiel 3:

Eine wie in Beispiel 1 behandelte organische Spaltproduktphase wurde in mehreren Destillationsschritten aufgearbeitet. Bei der destillativen Aufarbeitung wurde eine wäßrige Phase erhalten, welche die organischen Säuren, insbesondere Essig- und Ameisensäure, enthielt. Die wäßrige Phase wurde mit 225 l/h durch einen mit 15,3 l eines stark basischen Anionenaustauscherharzes (Amberjet 4400 OH, Firma Rohm und Haas) gefüllten Ionenaustauscherturms gefahren. Der Säuregehalt hatte sich nach der Behandlung mit dem Ionenaustauscher von durchschnittlich 1000-1500 ppm auf durchschnittlich < 25 ppm, berechnet als Essigsäure, verringert.

## Patentansprüche

1. Verfahren zur Entfernung von organischen und/oder anorganischen Säuren aus Spaltproduktphasen, die bei der Spaltung von Aralkylhydroperoxiden entstehen, durch Extraktion der Spaltproduktphasen mittels zumindest eines Extraktionsmittels und nachfolgender Behandlung des organischen Teils der Phasen mit einem Ionenaustauscher,
dadurch gekennzeichnet,
daß die mit einem Ionenaustauscher behandelten organischen Phasen zumindest einmal destillativ behandelt werden, bei dieser Behandlung zumindest eine überwiegend wäßrige, zumindest eine organische Säure aufweisende Phase von den organischen Phasen abgetrennt wird und diese Phase mit einem zweiten Ionenaustauscher behandelt wird.

2. Verfahren nach Anspruch 2,
dadurch gekennzeichnet,
daß die mit einem Ionenaustauscher behandelte organische Phase zumindest einmal in einer Destillationskolonne destilliert wird.

3. Verfahren nach zumindest einem der Ansprüche 1 oder 2,
dadurch gekennzeichnet,
daß zumindest eine überwiegend wäßrige, zumindest eine organische Säure aufweisende Phase, welche durch die zumindest einmalige destillative Behandlung der organischen Phase von dieser organischen Phase abgetrennt wird, mit einem stark basischen Anionenaustauscher behandelt wird.

4. Verfahren nach zumindest einem der Ansprüche 1 bis 3,
dadurch gekennzeichnet,
daß als Extraktionsmittel die überwiegend wäßrige Phase verwendet wird, die mit dem stark basischen Anionenaustauscher behandelt wurde.

5. Verfahren nach zumindest einem der Ansprüche 1 bis 4,
dadurch gekennzeichnet,
daß der Spaltproduktphase vor der Extraktion zumindest ein alkalischer Phenolstrom zugefügt wird.

6. Verfahren zur Entfernung von organischen und/oder anorganischen Säuren aus Spaltproduktphasen, die bei der Spaltung von Aralkylhydroperoxiden entstehen, durch Extraktion der Spaltproduktphasen mittels zumindest eines Extraktionsmittels und nachfolgender Behandlung des organischen Teils der Phasen mit einem Ionenaustauscher,
dadurch gekennzeichnet,
daß der Spaltproduktphase vor der Extraktion zumindest ein alkalischer Phenolstrom zugefügt wird.

7. Verfahren nach zumindest einem der Ansprüche 1 bis 5 oder nach Anspruch 6,
dadurch gekennzeichnet,
daß zur Extraktion bis zu 14 Gew.-% Wasser, bezogen auf die Spaltproduktphase, verwendet werden.

8. Verfahren nach zumindest einem der Ansprüche 1 bis 5 oder nach zumindest einem der Ansprüche 6 oder 7,
dadurch gekennzeichnet,
daß zur Extraktion einer mit Wasser gesättigten Spaltproduktphase zusätzlich bis zu 7 Gew.-% Wasser, bezogen auf die Spaltproduktphase, verwendet werden.

9. Verfahren nach Anspruch 8,
dadurch gekennzeichnet,
daß zur Extraktion einer mit Wasser gesättigten Spaltproduktphase zusätzlich von 2 bis 7 Gew.-% Wasser, bezogen auf die Spaltproduktphase, verwendet werden.

10. Verfahren nach Anspruch 9,
dadurch gekennzeichnet,
daß zur Extraktion einer mit Wasser gesättigten Spaltproduktphase zusätzlich von 2 bis 4 Gew.-% Wasser, bezogen auf die Spaltproduktphase, verwendet werden.

11. Verfahren nach zumindest einem der Ansprüche 1 bis 5 oder nach zumindest einem der Ansprüche 6 bis 10,
dadurch gekennzeichnet,
daß die Temperatur bei der Extraktion zwischen 10 und 90 °C liegt.

12. Verfahren nach Anspruch 11,
dadurch gekennzeichnet,
daß die Temperatur bei der Extraktion zwischen 20 und 50 °C liegt.

13. Verfahren nach Anspruch 12,
dadurch gekennzeichnet,
daß die Temperatur bei der Extraktion zwischen 30 und 40 °C liegt.

14. Verfahren nach zumindest einem der Ansprüche 1 bis 5 oder nach zumindest einem der Ansprüche 6 bis 13,
dadurch gekennzeichnet,
daß der nach der Extraktion erhaltene organische Teil der Spaltproduktphasen mit einem schwach basischen Anionenaustauscher behandelt wird.

15. Verfahren nach zumindest einem der Ansprüche 1 bis 5 oder nach zumindest einem der Ansprüche 6 bis 14,
dadurch gekennzeichnet,
daß der nach der Extraktion erhaltene organische Teil der Spaltproduktphasen vor der Behandlung mit einem schwach basischen Anionenaustauscher mit einem Koaleszer behandelt wird.

16. Verfahren nach zumindest einem der Ansprüche 1 bis 5 oder nach zumindest einem der Ansprüche 6 bis 15,
dadurch gekennzeichnet,
daß zur Regeneration der Anionenaustauscher Alkalihydroxid-Lösung verwendet wird.

17. Verfahren nach zumindest einem der Ansprüche 1 bis 5 oder nach zumindest einem der Ansprüche 6 bis 16,
dadurch gekennzeichnet,
daß zur Regeneration des schwach basischen Anionenaustauschers Phenolatlauge verwendet wird.

18. Verfahren nach zumindest einem der Ansprüche 1 bis 5 oder nach zumindest einem der Ansprüche 6 bis 17,
dadurch gekennzeichnet,
daß Spaltproduktphasen, die bei der Spaltung von Cumolhydroperoxid entstehen, verwendet werden.
